# EUROPEAN PATENT SPECIFICATION

(11) **EP 3 885 665 B1**
(45) Date of publication and mention of the grant of the patent: **13.08.2025**
(21) Application number: 20882527.3
(22) Date of filing: 22.04.2020
(51) Int. Cl.: A61N 1/40

(54) **SCHUMANN WAVE GENERATING APPARATUS AND WAVE MODULATION METHOD THEREFOR, AND AIR CONDITIONER**
SCHUMANN-WELLENGENERATOR UND WELLENMODULATIONSVERFAHREN DAFÜR SOWIE KLIMAANLAGE
APPAREIL DE GÉNÉRATION D'ONDE DE SCHUMANN ET PROCÉDÉ DE MODULATION D'ONDE ASSOCIÉ, ET CONDITIONNEUR D'AIR

(30) Priority: 31.10.2019 CN 201911055563
(43) Date of publication of application: 29.09.2021
(73) Proprietor: Haier Shenzen Research and Development Co., Ltd, Guangdong 518000 (CN); Haier Smart Home Co., Ltd., Qingdao, Shandong 266101 (CN)
(72) Inventor: LI, Xiang, Shenzhen, Guangdong 518000 (CN); WANG, Yongtao, Shenzhen, Guangdong 518000 (CN); ZHANG, Hui, Shenzhen, Guangdong 518000 (CN)
(74) Representative: dompatent von Kreisler Selting Werner - Partnerschaft von Patent- und Rechtsanwälten mbB
(86) International application number: PCT/CN2020/086065
(87) International publication number: WO 2021/082368

(56) References cited:
- EP-A2- 0 763 699
- CN-A- 1 649 254
- CN-A- 106 330 141
- CN-A- 106 931 517
- CN-A- 108 880 578
- CN-A- 109 078 266
- CN-A- 109 078 266
- CN-A- 109 547 049
- CN-A- 109 547 049
- JP-A- 2013 081 519
- JP-B2- 4 821 957
- TW-U- M 562 164
- US-A- 5 449 376
- US-A1- 2008 097 142
- US-A1- 2010 130 812
- US-A1- 2014 371 516

## Description

### CROSS-REFERENCE TO RELATED APPLICATIONS

The present application is based on and claims priority to Chinese Patent Application Serial No. 201911055563.X, filed on October 31, 2019.

### TECHNICAL FIELD

The present application relates to the field of electronic technologies, for example, to a Schumann wave generating apparatus, a wave modulation method therefor, and an air conditioner.

### BACKGROUND

A Schumann wave is an electromagnetic wave with an extremely low frequency existing in the earth, excited by lightning discharge, and has a wavelength approximately equal to a circumference of the earth. The frequency of the Schumann wave is controlled by earth ionospheric waveguide, a dominant frequency being about 7.83 Hz, and an alpha wave and a theta wave of a human brain have frequencies also close to 7.8 Hz. Therefore, when reaching the human body, the Schumann wave may harmoniously resonate with a physiological rhythm of a person to assist a running rhythm of a human organ, thereby relaxing the person. Currently, a Schumann wave generator on the market includes a modulation signal circuit and may generate a Schumann wave having a frequency of about 7.83 Hz and possessing amplitude and frequency which change regularly.

In the process of implementing embodiments of the present disclosure, a related art at least has the following problem: due to an influence of reflection of the previously generated Schumann wave, a Schumann wave signal generated by the existing Schumann wave generator is not clear enough, which is unable to well relax the human organ. Document JP4821957B2 discloses an apparatus according to the preamble of claim 1.

**SUMMARY** The invention is defined in claims 1 and 6.

A brief summary is given below to provide a basic understanding of some aspects of the embodiments disclosed. The section of summary is not to provide a general commentary, or to determine the key/important elements or to describe the protection scopes of these embodiments, but acts as a preamble of the subsequent detailed illustration.

Embodiments of the present disclosure provide a Schumann wave generating apparatus, a wave modulation method therefor, and an air conditioner, so as to solve the technical problem that a Schumann wave signal generated by an existing Schumann wave generator is not clear enough and a human organ cannot be well relaxed.

In some embodiments, an air conditioner includes the a Schumann wave generating apparatus according to claim 1.

The Schumann wave generating apparatus, the wave modulation method therefor, and the air conditioner according to the embodiments of the present disclosure may realize the following technical effects.

According to the Schumann wave signal received by the receiving module, the modulation module is controlled to adjust the generation state of the Schumann wave, so as to reduce the influence of reflection of a previously generated Schumann wave, such that the Schumann wave signal in space is clearer and then better relaxes the human organ.

The foregoing general description and the following description are merely exemplary and explanatory and are not restrictive of the present application.

### BRIEF DESCRIPTION OF THE DRAWINGS

One or more embodiments are illustrated in the accompanying drawings corresponding thereto, these illustrations and drawings do not constitute a limitation on the embodiments, elements with the same reference numerals in the drawings representing similar elements, the drawings do not constitute a scale limitation, and in the drawings:
Figure 1 is a schematic structural diagram of a Schumann wave generating apparatus according to embodiments of the present disclosure;
Figure 2 is a circuit diagram of a control module according to embodiments of the present disclosure;
Figure 3 is a circuit diagram of a receiving module according to embodiments of the present disclosure;
Figure 4 is a circuit diagram of a driving circuit according to embodiments of the present disclosure;
Figure 5 is a circuit diagram of a boost circuit according to embodiments of the present disclosure;
Figure 6 is a schematic flow chart of a wave modulation method for a Schumann wave generating apparatus according to embodiments of the present disclosure;
Figure 7 is a schematic flow chart of a wave modulation method for a Schumann wave generating apparatus according to embodiments of the present disclosure; and
Figure 8 is a schematic structural diagram of a wave modulation apparatus for a Schumann wave generating apparatus according to embodiments of the present disclosure.

### DETAILED DESCRIPTION

In order to have a more detailed understanding of the features and technical content of the embodiments of the present disclosure, the implementation of the embodiments of the present disclosure will be described in detail below with reference to the accompanying drawings. The attached drawings are for reference only and are not used to limit the embodiments of the present disclosure. In the following technological description, for purposes of explanation, numerous details are set forth in order to provide a thorough understanding of the disclosed embodiments. However, one or more embodiments may be practiced without these details. In other instances, well-known structures and apparatuses may be shown in simplified forms in order to simplify the drawings.

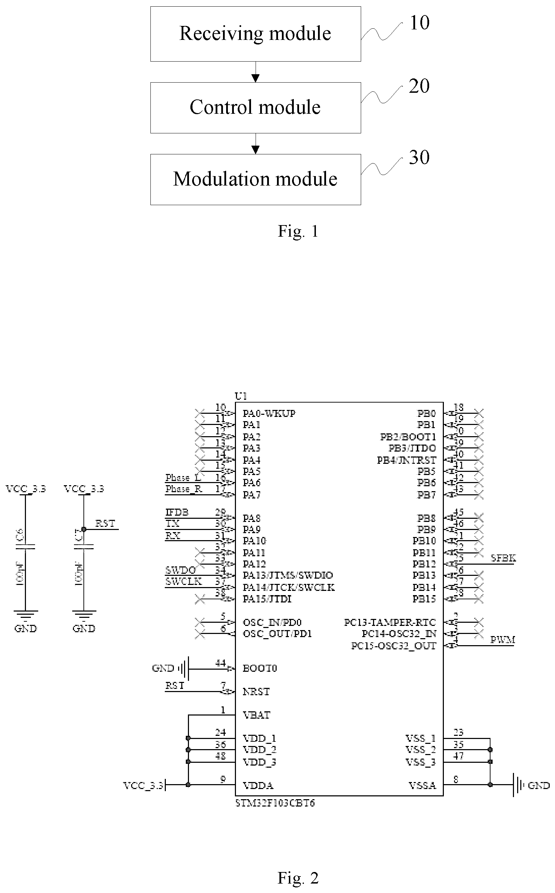

The present disclosure provides a Schumann wave generating apparatus including a receiving module 10, a modulation module 30, and a control module 20, as shown in Figure 1. The receiving module 10 is configured to receive a Schumann wave signal; the modulation module 30 is configured to modulate a generation state of a Schumann wave; and the control module 20 is configured to control, according to the Schumann wave signal received by the receiving module 10, the modulation module 30 to adjust the generation state of the Schumann wave.

Optionally, a preset condition is as follows: the received Schumann wave signal is the same as a transmitted Schumann wave signal. When having the same waveform as the transmitted Schumann wave signal, the received Schumann wave signal is determined to be the same as the transmitted Schumann wave signal. If reflected in space, a Schumann wave previously generated by the Schumann wave generating apparatus may interfere with the waveform of the Schumann wave currently generated by the Schumann wave generating apparatus, such that the received and transmitted Schumann wave signals have different waveforms; and if not reflected in space, that is, energy of the Schumann wave being exhausted in the propagation process, the Schumann wave previously generated by the Schumann wave generating apparatus may not interfere with the waveform of the Schumann wave currently generated by the Schumann wave generating apparatus, such that the received Schumann wave signal has the same waveform as the transmitted Schumann wave signal.

When the received Schumann wave signal meets the preset condition, the control module 20 controls the modulation module 30 to increase a generation amplitude of the Schumann wave; and when the received Schumann wave signal does not meet the preset condition, the control module 20 controls the modulation module 30 to increase a generation interval of the Schumann wave. When the received Schumann wave signal meets the preset condition, the Schumann wave currently generated by the Schumann wave generating apparatus is less influenced by the reflection of the previously generated Schumann wave signal, and the generation amplitude of the Schumann wave signal is increased properly to improve a resonance strength of a human organ and the Schumann wave signal, thus better assisting the running rhythm of the human organ; and when the received Schumann wave signal does not meet the preset condition, the Schumann wave currently generated by the Schumann wave generating apparatus is influenced by the reflection of the previously generated Schumann wave signal, and the generation interval of the Schumann wave signal is increased properly to increase the energy dissipation duration of the previously generated Schumann wave until the generated Schumann wave signal is not influenced by the reflection of the previously generated Schumann wave signal, such that the Schumann wave signal in space is clearer to better assist the resonance of the human organ.

In the embodiments of the present disclosure, according to the Schumann wave signal received by the receiving module 10, the modulation module 30 is controlled to adjust the generation state of the Schumann wave, so as to reduce the influence of the reflection of the previously generated Schumann wave, such that the Schumann wave signal in space is clearer and then better relaxes the human organ.

In some embodiments, as shown in Figure 2, an STM32 single chip microcomputer is used as the control module 20. Available modes of the STM32 single chip microcomputer include low power consumption modes, such as a low-power-consumption operation mode, a low-power-consumption sleep mode, a shutdown mode, a standby mode, or the like, and a better balance point may be achieved among low power consumption, short startup time and available wake-up sources, thereby significantly reducing the power consumption of the control module 20.

Optionally, the STM32 single chip microcomputer has a supply voltage of 3.3V (volts). The operating time of a battery powered device may be prolonged when a supply voltage is reduced.

In some embodiments, the receiving module 10 includes a receiving circuit configured to receive the Schumann wave signal in preset space; and a filter circuit provided to be electrically connected with the receiving circuit and configured to filter the Schumann wave signal received by the receiving circuit.

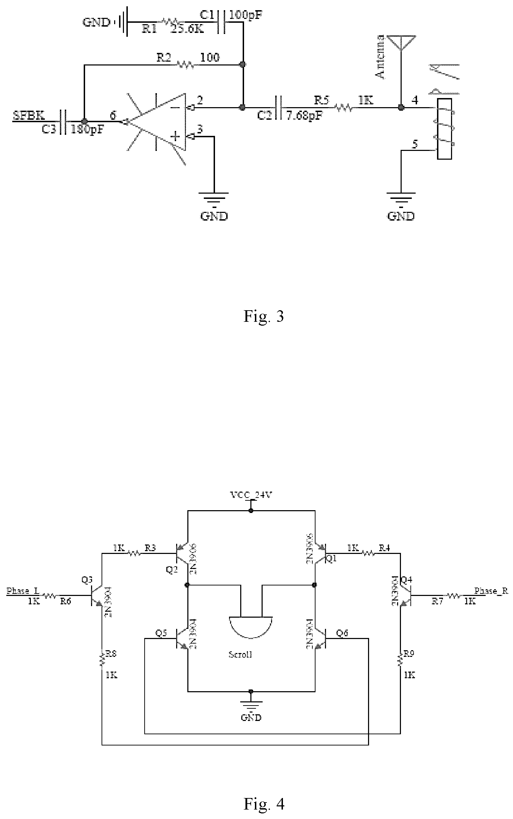

Optionally, as shown in Figure 3, the receiving circuit includes an antenna, a fifth resistor R5, and a second capacitor C2, wherein the fifth resistor R5 has a first end connected with the antenna and a second end connected with a first end of the second capacitor C2, and the second capacitor C2 has a second end connected with the filter circuit.

Optionally, as shown in Figure 3, the filter circuit includes a first resistor R1, a second resistor R2, a first capacitor C1, a third capacitor C3, and a first operational amplifier, wherein the first resistor R1 has a first end which is grounded GND, and a second end connected with a first end of the first capacitor C1; the first capacitor C1 has a second end connected with a second end of the second capacitor C2; the second resistor R2 has a first end connected with a first end of the third capacitor C3 and a second end connected with the second end of the second capacitor C2; the first operational amplifier has a first end connected with the first end of the third capacitor C3, a second end connected with the second end of the second capacitor C2, and a third end which is grounded GND; and the third capacitor has a second end connected with the single chip microcomputer.

The antenna in the receiving circuit receives the Schumann wave signal in space, the signal is filtered by a band-pass filter formed by the filter circuit, and then, the processed Schumann wave signal is transferred to the single chip microcomputer for arbitration, so as to judge whether the received Schumann wave signal meets the preset condition or not. The received Schumann wave signal is filtered to remove other interference signals in space, such that the influence of irrelevant signals on the judgment result of the Schumann wave signal may be reduced to improve the accuracy of the arbitration result made by the single chip microcomputer.

In some embodiments, the modulation module 30 includes a Schumann wave generator; a driving circuit provided to be electrically connected with the Schumann wave generator and configured to drive the Schumann wave generator to generate a Schumann wave with an adjustable frequency; and a boost circuit configured to be electrically connected with the Schumann wave generator and adjust the amplitude of the Schumann wave generated by the Schumann wave generator.

Optionally, as shown in Figure 4, the driving circuit includes a first driving circuit and a second driving circuit which are disposed symmetrically. The first driving circuit includes a third resistor R3, a sixth resistor R6, an eighth resistor R8, a second triode Q2, a third triode Q3 and a fifth triode Q5, wherein the sixth resistor R6 has a first end connected with the single chip microcomputer and a second end connected with a first end of the third triode Q3; the third resistor R3 has a first end connected with a second end of the third triode Q3 and a second end connected with a first end of the second triode Q2; the eighth resistor R8 has a first end connected with a third end of the third triode Q3 and a second end connected with the second driving circuit; the second triode Q2 has a second end connected with a power source (VCC_24V) and a third end connected with the Schumann wave generator Scroll; and the fifth triode Q5 has a first end connected with the second driving circuit, a second end connected with the Schumann wave generator Scroll, and a third end which is grounded GND. The second driving circuit includes a fourth resistor R4, a seventh resistor R7, a ninth resistor R9, a first triode Q1, a fourth triode Q4 and a sixth triode Q6, wherein the seventh resistor R7 has a first end connected with the single chip microcomputer and a second end connected with a first end of the fourth triode Q4; the fourth resistor R4 has a first end connected with a second end of the fourth triode Q4 and a second end connected with a first end of the first triode Q1; the ninth resistor R9 has a first end connected with a third end of the fourth triode Q4 and a second end connected with the first end of the fifth triode Q5; the first triode Q1 has a second end connected with the power source (VCC_24V) and a third end connected with the Schumann wave generator Scroll; and the sixth triode Q6 has a first end connected with the second end of the eighth resistor R8, a second end connected with the Schumann wave generator Scroll, and a third end which is grounded GND.

The driving circuit is implemented by a bidirectional bridge circuit, and the single chip microcomputer controls two bridge arms by controlling Phase_L and Phase_R, so as to drive the Schumann wave generator Scroll to generate the Schumann wave with an adjustable frequency. When the single chip microcomputer controls Phase_L to output a high level and Phase_R to output a low level, the fourth triode Q4 is turned off, and a current flows from the power source (VCC_24V) to the Schumann wave generator Scroll through the second triode Q2 and then from the sixth triode Q6 to GND; and when the single chip microcomputer controls Phase_L to output a low level and Phase_R to output a high level, the third triode Q3 is turned off, and a current flows from the power source (VCC_24V) to the Schumann wave generator Scroll through the first triode Q1 and then from the fifth triode Q5 to GND. In both states, the currents flowing through the Schumann wave generator Scroll are opposite, such that alternating voltages are generated at two ends of the Schumann wave generator Scroll, so as to drive the Schumann wave generator Scroll to generate a Schumann wave signal with an adjustable frequency.

In some embodiments, the boost circuit includes a boost subcircuit configured to adjust the amplitude of the Schumann wave generated by the Schumann wave generator; and a detection subcircuit provided to be connected with the boost subcircuit and configured to detect an operating current of the boost subcircuit.

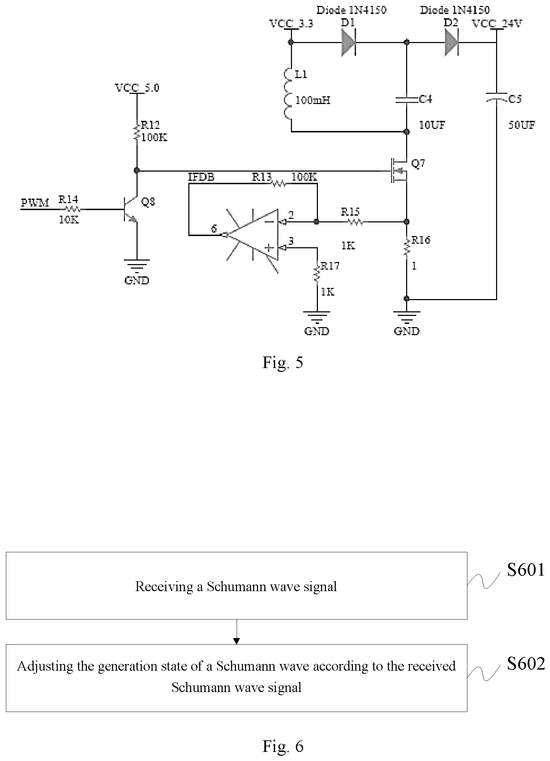

Optionally, as shown in Figure 5, the boost subcircuit includes a twelfth resistor R12, a fourteenth resistor R14, a sixteenth resistor R16, a first inductor L1, a fourth capacitor C4, a fifth capacitor C5, a first diode D1, a second diode D2, a seventh triode Q7, and an eighth triode Q8, wherein the fourteenth resistor R14 has a first end connected with the single chip microcomputer and a second end connected with a first end of the eighth triode Q8; the twelfth resistor R12 has a first end connected with a power source (VCC_5.0V) and a second end connected with a second end of the eighth triode Q8; the sixteenth resistor R16 has a first end connected with a third end of the seventh triode Q7 and a second end which is grounded GND; the first inductor L1 has a first end connected with a power source (VCC_3.3V) and a second end connected with a second end of the seventh triode Q7; the fourth capacitor C4 has a first end connected with a second end of the first diode D1 and a second end connected with the second end of the seventh triode Q7; the fifth capacitor C5 has a first end connected with the power source (VCC_24V) and a second end which is grounded GND; the first diode D1 has a first end connected with the power source (VCC_3.3V); the second diode D2 has a first end connected with the second end of the first diode D1; the seventh triode Q7 has a first end connected with the second end of the eighth triode Q8; and the eighth triode Q8 has a third end which is grounded GND.

The single chip microcomputer performs control to output a PWM square wave to control the booster subcircuit, so as to adjust the amplitude of the Schumann wave generated by the Schumann wave generator Scroll. When a PWM signal output under the control of the single chip microcomputer is at a high level, a voltage output to the seventh triode Q7 is at a low level under the inversion action of the eighth triode Q8, and the seventh triode Q7 is turned off; and when the PWM signal output under the control of the single chip microcomputer is at a low level, the eighth triode Q8 is turned off, the voltage output to the seventh triode Q7 is at a high level under the inversion action of the eighth triode Q8, and the seventh triode Q7 is turned on. When the seventh triode Q7 is turned on, a current may be supplied from the power source VCC_3.3V to charge the fourth and fifth capacitors C4, C5; and when the seventh triode Q7 is turned off, the current is unable to change instantaneously due to the first inductor L1, the first inductor L1 continues to charge the fourth and fifth capacitors C4, C5, and a certain voltage exists across each of the fourth and fifth capacitors C4, C5 which are fully charged. The higher the proportion of the low level in the PWM signal output under the control of the single chip microcomputer, the longer the ON time of the seventh triode Q7, the longer the charging time of the fourth and fifth capacitors C4, C5, and thus, the higher the voltage across each of the fourth and fifth capacitors C4, C5. The voltage across each of the fourth and fifth capacitors C4, C5 is an adjustable voltage for driving the Schumann wave generator Scroll, and the amplitude of the Schumann wave generated by the Schumann wave generator may be adjusted by adjusting a voltage value for driving the Schumann wave generator Scroll.

Optionally, as shown in Figure 5, the detection subcircuit includes a thirteenth resistor R13, a fifteenth resistor R15, a seventeenth resistor R17 and a second operational amplifier, wherein the thirteenth resistor R13 has a first end connected with a first end of the second operational amplifier and a second end connected with a second end of the second operational amplifier; the fifteenth resistor R15 has a first end connected with the second end of the second operational amplifier and a second end connected with a first end of the sixteenth resistor R16; the seventeenth resistor R17 has a first end connected with a third end of the second operational amplifier and a second end which is grounded GND; and the first end of the second operational amplifier is connected with the single chip microcomputer.

The purpose of detecting the operating current of the boost subcircuit is achieved by detecting the voltage of the sixteenth resistor R16. A voltage signal processed by the second operational amplifier is transmitted to the single chip microcomputer, and when the voltage signal is greater than a preset voltage signal, the operating current of the boost subcircuit is over-large, such that effective actions are taken to reduce the operating current of the boost subcircuit and guarantee normal and stable operation thereof.

In some embodiments, the Schumann wave generating apparatus further includes a display module provided to be electrically connected with the control module and configured to display the frequency and amplitude of the Schumann wave. Optionally, the display module includes a mobile phone. Thus, a user may learn the operating state of the Schumann wave generating apparatus conveniently.

The embodiments of the present disclosure further provide a wave modulation method for a Schumann wave generating apparatus which, as shown in Figure 6, includes the following steps:
S601: receiving a Schumann wave signal.

Optionally, the Schumann wave signal in space is received by a receiving module of the Schumann wave generating apparatus.

S602: adjusting the generation state of a Schumann wave according to the received Schumann wave signal.

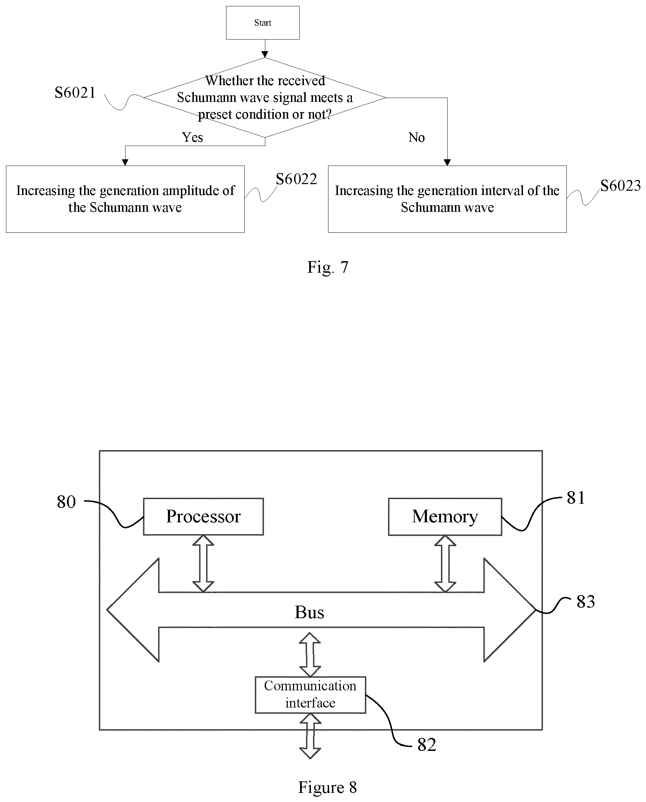

In some embodiments, as shown in Figure 7, the adjusting the generation state of a Schumann wave according to the received Schumann wave signal includes the following steps:
S6021: judging whether the received Schumann wave signal meets a preset condition or not.
S6022: increasing the generation amplitude of the Schumann wave when the received Schumann wave signal meets the preset condition.
S6023: increasing the generation interval of the Schumann wave when the received Schumann wave signal does not meet the preset condition.

In the embodiments of the present disclosure, the generation state of the Schumann wave is adjusted according to the received Schumann wave signal, so as to reduce the influence of the reflection of the previously generated Schumann wave, such that the Schumann wave signal in space is clearer and then better relaxes the human organ.

The embodiments of the present disclosure further provide a wave modulation apparatus for a Schumann wave generating apparatus which, as shown in Figure 8, includes:
a processor 80 and a memory 81, and may further include a communication interface 82 and a bus 83. The processor 80, the communication interface 82 and the memory 81 may be communicated with one another through the bus 83. The communication interface 82 may be used for information transmission. The processor 80 may call logic instructions in the memory 81 to perform the wave modulation method for a Schumann wave generating apparatus according to the above-mentioned embodiment.

In addition, the above-mentioned logic instructions in the memory 81 may be implemented in the form of software functional units and stored in a computer-readable storage medium when sold or used as independent products.

The memory 81 may be used as a computer-readable storage medium for storing software programs and computer-executable programs, such as program instructions/modules corresponding to the methods according to the embodiments of the present disclosure. The processor 80 executes functional applications and data processing operations by running the program instructions/modules stored in the memory 81, that is, implements the wave modulation method for a Schumann wave generating apparatus in the above-mentioned method embodiment.

The memory 81 may include a program storage area for storing an operating system and an application program required by at least one function; and a data storage area for storing data created according to usage of a terminal device, or the like. Furthermore, the memory 81 may include a high-speed random access memory and may also include a nonvolatile memory.

The embodiments of the present disclosure further provide an air conditioner including the above-mentioned Schumann wave generating apparatus.

The embodiments of the present disclosure provide a computer-readable storage medium for storing computer-executable instructions configured to execute the above-mentioned wave modulation method for a Schumann wave generating apparatus.

The embodiments of the present disclosure provide a computer program product including a computer program stored on a computer-readable storage medium, the computer program including program instructions which, when executed by a computer, cause the computer to perform the above-mentioned wave modulation method for a Schumann wave generating apparatus.

The above-mentioned computer-readable storage medium may be a transitory computer-readable storage medium or a non-transitory computer-readable storage medium.

The technical solution of the embodiments of the present disclosure may be embodied in the form of a software product, which is stored in a storage medium and includes one or more instructions for causing a computer device (which may be a personal computer, a server, a network device, or the like) to execute all or part of the steps of the method according to the embodiments of the present disclosure. The aforementioned storage medium may be a non-transitory storage medium including: a USB flash disk, a mobile hard disk, a Read-Only Memory (ROM), a Random Access Memory (RAM), a magnetic disk, an optical disk, and other media capable of storing program codes, and may also be a transient storage medium.

The above description and accompanying drawings fully illustrate the embodiments of the present disclosure, to enable one skilled in the art to implement the embodiments. Modifications, such as structural, logical, electrical, process and other modifications, can be made in other embodiments. The embodiments only represent some possible variations. Individual components or functions are optional and the operation order is variable, unless it is otherwise stated specifically. A part and certain feature of some embodiments may be included in or replaced by a part and certain feature of other embodiments. The scope of the embodiments of the present disclosure includes the whole scope of the claims and all obtainable equivalents thereof. As used in the present application, although the terms "first", "second", etc. may be used herein to describe various elements, these elements should not be limited by these terms. These terms are only used to distinguish one element from another. For example, without changing the meaning of the description, a first element could be termed a second element, and similarly, a second element could be termed a first element, provided that all the first elements are renamed consistently and all the second elements are renamed consistently. The first and second elements are both elements, but may not be the same elements. The terminology used herein is for the purpose of describing the embodiments only and is not intended to be limiting of the claims.

In a case of no more limitations, elements limited by a sentence of "includes a . . ." do not exclude that other same elements further exist in the procedure, the method, or the equipment including the elements. In the present specification, description of each of the embodiments may focus on differences from other embodiments, and reference may be made to each other for the same or similar parts among respective embodiments. If the method, product, or the like, according to the embodiments correspond to the method according to the embodiments, reference may be made to the method description for the associated part.

Those skilled in the art may be aware that, in combination with the examples described in the embodiments disclosed in this specification, units and algorithm steps may be implemented by electronic hardware or a combination of computer software and electronic hardware. Whether the functions are performed by hardware or software depends on particular applications and design constraint conditions of the technical solutions. A person skilled in the art may use different methods to implement the described functions for each particular application, but it should not be considered that the implementation goes beyond the scope of the embodiments of the present disclosure. It may be clearly understood by a person skilled in the art that, for the purpose of convenient and brief description, for a detailed working process of the foregoing system, apparatus, and unit, reference may be made to a corresponding process in the foregoing method embodiments, and details are not described herein again.

In the embodiments disclosed herein, the disclosed method and product (including, but being not limited to, an apparatus, a device, or the like) may be implemented in other manners. For example, the described apparatus embodiment is merely an example. For example, the unit division is merely logical function division and may be other division in actual implementation. For example, a plurality of units or components may be combined or integrated into another system, or some features may be ignored or not performed. In addition, the displayed or discussed mutual couplings or direct couplings or communication connections may be implemented by using some interfaces. The indirect couplings or communication connections between the apparatuses or units may be implemented in electronic, mechanical, or other forms. The units described as separate parts may or may not be physically separate, and parts displayed as units may or may not be physical units, may be located in one position, or may be distributed on a plurality of network units. Some or all of the units may be selected according to actual needs to implement the embodiments. In addition, functional units in the embodiments of the present disclosure may be integrated into one processing unit, or each of the units may exist alone physically, or two or more units are integrated into one unit.

The flowchart and block diagrams in the figures illustrate the architecture, functionality, and operation of possible implementations of systems, methods and computer program products according to the embodiments of the present disclosure. In this regard, each block in the flowchart or block diagrams may represent a module, segment, or portion of code, which comprises one or more executable instructions for implementing the specified logical function(s). In some alternative implementations, the functions noted in the block may occur out of the order noted in the figures. For example, two blocks shown in succession may, in fact, be executed substantially concurrently, or the blocks may sometimes be executed in the reverse order, depending upon the functionality involved. In the description corresponding to the flowchart and block diagrams in the figures, operations or steps corresponding to different blocks may also occur out of the order disclosed in the description, and sometimes, there is no specific order between different operations or steps. For example, two operations or steps shown in succession may, in fact, be executed substantially concurrently, or the operations or steps may sometimes be executed in the reverse order, depending upon the functionality involved. Each block of the block diagrams and/or flowcharts, and combinations of blocks in the block diagrams and/or flowcharts, can be implemented by special purpose hardware-based systems that perform the specified functions or acts, or combinations of special purpose hardware and computer instructions.

## Claims

1. A Schumann wave generating apparatus, comprising:
a receiving module (10) configured to receive a Schumann wave signal;
a modulation module (30) configured to modulate a generation state of a Schumann wave; and
a control module (20) configured to control, according to the Schumann wave signal received by the receiving module, the modulation module to adjust the generation state of the Schumann wave **characterized in that** the control module controls the modulation module to increase the generation amplitude of the Schumann wave when the received Schumann wave signal meets a preset condition; and
the control module controls the modulation module to increase the generation interval of the Schumann wave when the received Schumann wave signal does not meet the preset condition.

2. The Schumann wave generating apparatus according to claim 1, wherein the preset condition comprises that:
the received Schumann wave signal is the same as a transmitted Schumann wave signal.

3. The Schumann wave generating apparatus according to claim 1 or 2, wherein the receiving module comprises:
a receiving circuit configured to receive the Schumann wave signal in preset space; and
a filter circuit provided to be electrically connected with the receiving circuit and configured to filter the Schumann wave signal received by the receiving circuit.

4. The Schumann wave generating apparatus according to any one of claims 1 to 3, wherein the modulation module comprises:
a Schumann wave generator;
a driving circuit provided to be electrically connected with the Schumann wave generator and configured to drive the Schumann wave generator to generate a Schumann wave with an adjustable frequency; and
a boost circuit configured to be electrically connected with the Schumann wave generator and adjust the amplitude of the Schumann wave generated by the Schumann wave generator.

5. The Schumann wave generating apparatus according to claim 4, wherein the boost circuit comprises:
a boost subcircuit configured to adjust the amplitude of the Schumann wave generated by the Schumann wave generator; and
a detection subcircuit provided to be connected with the boost subcircuit and configured to detect an operating current of the boost subcircuit.

6. A wave modulation method for a Schumann wave generating apparatus, comprising:
receiving a Schumann wave signal; and
adjusting the generation state of a Schumann wave according to the received Schumann wave signal,
**characterized in that**
the adjusting the generation state of a Schumann wave according to the received Schumann wave signal comprises:
increasing the generation amplitude of the Schumann wave when the received Schumann wave signal meets a preset condition; and
increasing the generation interval of the Schumann wave when the received Schumann wave signal does not meet the preset condition.

7. The wave modulation method according to claim 6, wherein the preset condition comprises that:
the received Schumann wave signal is the same as a transmitted Schumann wave signal.

8. An air conditioner, comprising the Schumann wave generating apparatus according to any one of claims 1 to 5.

## Patentansprüche

1. Schumann-Wellengeneratorvorrichtung mit:
einem Empfangsmodul (10), das zum Empfangen eines Schumann-Wellensignals ausgebildet ist;
einem Modulationsmodul (30), das zum Modulieren eines Erzeugungszustands einer Schumann-Welle ausgebildet ist; und
einem Steuermodul (20), das dazu ausgebildet ist, das Modulationsmodul gemäß dem von dem Empfangsmodul empfangenen Schumann-Wellensignal zu steuern, um den Erzeugungszustand der Schumann-Welle anzupassen,
**dadurch gekennzeichnet, dass** das Steuermodul das Modulationsmodul zum Erhöhen der Amplitude der Schumann-Welle steuert, wenn das empfangene Schumann-Wellensignal eine voreingestellte Bedingung erfüllt; und
das Steuermodul das Modulationsmodul zum Vergrößern des Erzeugungsintervalls der Schumann-Welle steuert, wenn das empfangene Schumann-Wellensignal die voreingestellte Bedingung nicht erfüllt.

2. Schumann-Wellengeneratorvorrichtung nach Anspruch 1, bei welcher die voreingestellte Bedingung umfasst, dass das empfangene Schumann-Wellensignal dasselbe wie ein gesendetes Schumann-Wellensignal ist.

3. Schumann-Wellengeneratorvorrichtung nach Anspruch 1 oder 2, bei welcher das Empfangsmodul aufweist:
eine Empfangsschaltung, die dazu ausgebildet ist, das Schumann-Wellensignal in einem voreingestellten Raum zu empfangen; und
eine Filterschaltung, die zum elektrischen Verbinden mit der Empfangsschaltung vorgesehen ist und dazu ausgebildet ist, das von der Empfangsschaltung empfangene Schumann-Wellensignal zu filtern.

4. Schumann-Wellengeneratorvorrichtung nach einem der Ansprüche 1 bis 3, bei welcher das Modulationsmodul aufweist:
einen Schumann-Wellengenerator;
eine Ansteuerschaltung, die zum elektrischen Verbinden mit dem Schumann-Wellengenerator vorgesehen ist und dazu ausgebildet ist, den Schumann-Wellengenerator zum Erzeugen einer Schumann-Welle mit einstellbarer Frequenz anzusteuern; und
eine Boost-Schaltung, die zum elektrischen Verbinden mit dem Schumann-Wellengenerator und zum Einstellen der Amplitude der von dem Schumann-Wellengenerator erzeugten Schumann-Welle ausgebildet ist.

5. Schumann-Wellengeneratorvorrichtung nach Anspruch 4, bei welcher die Boost-Schaltung aufweist:
eine Boost-Unterschaltung, die zum Einstellen der Amplitude der von dem Schumann-Wellengenerator erzeugten Schumann-Welle ausgebildet ist; und
eine Erkennungsunterschaltung, die zum Verbinden mit der Boost-Unterschaltung vorgesehen ist und dazu ausgebildet ist, einen Betriebsstrom der Boost-Unterschaltung zu erkennen.

6. Wellenmodulationsverfahren für eine Schumann-Wellengeneratorvorrichtung mit den folgenden Schritten:
Empfangen eines Schumann-Wellensignals; und
Einstellen des Erzeugungszustands einer Schumann-Welle gemäß dem empfangenen Schumann-Wellensignal,
**dadurch gekennzeichnet, dass** das Einstellen des Erzeugungszustands einer Schumann-Welle gemäß dem empfangenen Schumann-Wellensignal die folgenden Schritte aufweist:
Erhöhen der Erzeugungsamplitude der Schumann-Welle, wenn das empfangene Schumann-Wellensignal eine voreingestellte Bedingung erfüllt; und
Vergrößern des Erzeugungsintervalls der Schumann-Welle, wenn das empfangene Schumann-Wellensignal nicht die voreingestellte Bedingung erfüllt.

7. Wellenmodulationsverfahren nach Anspruch 6, bei welchem die voreingestellte Bedingung umfasst, dass das empfangene Schumann-Wellensignal dasselbe wie ein gesendetes Schumann-Wellensignal ist.

8. Klimaanlage mit der Schumann-Wellengeneratorvorrichtung nach einem der Ansprüche 1 bis 5.

## Revendications

1. Appareil de génération d'onde de Schumann, comprenant :
un module de réception (10) configuré pour recevoir un signal d'onde de Schumann ;
un module de modulation (30) configuré pour moduler un état de génération d'une onde de Schumann ; et
un module de commande (20) configuré pour commander, conformément au signal d'onde de Schumann reçu par le module de réception, le module de modulation pour régler l'état de génération de l'onde de Schumann,
**caractérisé en ce que** le module de commande commande le module de modulation pour augmenter l'amplitude de génération de l'onde de Schumann lorsque le signal d'onde de Schumann reçu satisfait une condition prédéfinie ; et
le module de commande commande le module de modulation pour augmenter l'intervalle de génération de l'onde de Schumann lorsque le signal d'onde de Schumann reçu ne satisfait pas la condition prédéfinie.

2. Appareil de génération d'onde de Schumann selon la revendication 1, dans lequel la condition prédéfinie est telle que :
le signal d'onde de Schumann reçu est le même qu'un signal d'onde de Schumann transmis.

3. Appareil de génération d'onde de Schumann selon la revendication 1 ou la revendication 2, dans lequel le module de réception comprend :
un circuit de réception configuré pour recevoir le signal d'onde de Schumann dans un espace prédéfini ; et
un circuit de filtre prévu pour être connecté électriquement au circuit de réception et configuré pour filtrer le signal d'onde de Schumann reçu par le circuit de réception.

4. Appareil de génération d'onde de Schumann selon l'une quelconque des revendications 1 à 3, dans lequel le module de modulation comprend :
un générateur d'onde de Schumann ;
un circuit de pilotage prévu pour être connecté électriquement au générateur d'onde de Schumann et configuré pour piloter le générateur d'onde de Schumann pour générer une onde de Schumann ayant une fréquence réglable ; et
un circuit amplificateur configuré pour être connecté électriquement au générateur d'onde de Schumann et pour régler l'amplitude de l'onde de Schumann générée par le générateur d'onde de Schumann.

5. Appareil de génération d'onde de Schumann selon la revendication 4, dans lequel le circuit amplificateur comprend :
un sous-circuit amplificateur configuré pour régler l'amplitude de l'onde de Schumann générée par le générateur d'onde de Schumann ; et
un sous-circuit de détection prévu pour être connecté au sous-circuit amplificateur et configuré pour détecter un courant de fonctionnement du sous-circuit amplificateur.

6. Procédé de modulation d'onde pour un appareil de génération d'onde de Schumann, comprenant les étapes consistant à :
recevoir un signal d'onde de Schumann ; et
régler l'état de génération d'une onde de Schumann conformément au signal d'onde de Schumann reçu,
**caractérisé en ce que** l'étape de réglage de l'état de génération d'une onde de Schumann conformément au signal d'onde de Schumann reçu consiste à :
augmenter l'amplitude de génération de l'onde de Schumann lorsque le signal d'onde de Schumann reçu satisfait une condition prédéfinie ; et
augmenter l'intervalle de génération de l'onde de Schumann lorsque le signal d'onde de Schumann reçu ne satisfait pas la condition prédéfinie.

7. Procédé de modulation d'onde selon la revendication 6, dans lequel la condition prédéfinie est telle que :
le signal d'onde de Schumann reçu est le même qu'un signal d'onde de Schumann transmis.

8. Conditionneur d'air, comprenant l'appareil de génération d'onde de Schumann selon l'une quelconque des revendications 1 à 5.
